# NEW EUROPEAN PATENT SPECIFICATION

(11) **EP 2 098 499 B2**
(45) Date of publication and mention of the opposition decision: **22.11.2017**
(45) Mention of the grant of the patent: 03.09.2014
(21) Application number: 09154367.8
(22) Date of filing: 04.03.2009
(51) Int. Cl.: C07C 17/386, C07C 21/18

(54) **Azeotrope-like composition of 2-chloro-3,3,3-trifluoropropene (HCFC-1233xf) and hydrogen fluoride (HF)**
Azeotrop-ähnliche Zusammensetzung aus 2-Chlor-3,3,3,-Trifluorpropen (HCFC-1233xf) und Wasserstofffluorid (HF)
Composition de type azéotrope de 2-chloro-3,3,3-tétrafluoropropène (hcfc-1233xf) et fluorure d'hydrogène (HF)

(30) Priority: 06.03.2008 US 34184 P; 03.03.2009 US 396672; 03.03.2009 US 396528
(43) Date of publication of application: 09.09.2009
(73) Proprietor: Honeywell International Inc., Morristown, NJ 07962 (US)
(72) Inventor: Pham, Hang, T., Amherst, NY 14228 (US); Merkel, Daniel, C., West Seneca, NY 14224 (US); Pokrovski, Konstantin, A., Orchard Park, NY 14127 (US); Tung, HsuehSung, Getzville, NY 14068 (US); Singh, Rajiv, R., Getzville, NY 14068 (US)
(74) Representative: Gill Jennings & Every LLP

(56) References cited:
- EP-A- 0 940 382
- WO-A-2008/054781
- WO-A2-2007/053736
- WO-A2-2011/126634
- US-A1- 2003 096 724
- US-A1- 2007 007 488
- US-B2- 6 524 496
- "6.11 Wilson, NRTL, and UNIQUAC Equations", Prausnitz, Lichtenhalter, de Azevedo, Molecular Thermodnamics of Fluid-Phase Equilibria, 3rd Edition, Prentice-Hall, 1999, pages 258-264,287.
- RENON H. ET AL: 'Local compositions in thermodynamic excess functions for liquid mixtures' AICHE JOURNAL vol. 14, no. 1, 1968, pages 135 - 144
- SMITH J.M. ET AL: 'Introduction to Chemical Engineering Thermodynamics', vol. 5TH ED., THE MCGRAW-HILL COMPANIES, INC., NEW YORK pages 516 - 519

## Description

### BACKGROUND OF THE INVENTION

### FIELD OF THE INVENTION

The present invention pertains to azeotropic and azeotrope-like compositions of 2-chloro-3,3,3-trifluoropropene (HCFO-1233xf) and hydrogen fluoride (HF). More particularly the invention pertains to such azeotropic and azeotrope-like compositions which are useful as intermediates in the production of 2,3,3,3-tetrafluoropropene (HFO-1234yf).

### DESCRIPTION OF THE PRIOR ART

Traditionally, chlorofluorocarbons (CFCs) like trichlorofluoromethane and dichlorodifluoromethane have been used as refrigerants, blowing agents and diluents for gaseous sterilization. In recent years there has been universal concern that completely halogenated chlorofluorocarbons might be detrimental to the Earth's ozone layer. Therefore, stratospherically safer alternatives to these materials are desirable. Consequently, there is a worldwide effort to use fluorine-substituted hydrocarbons which contain fewer or no chlorine substituents. In this regard, 2-chloro-3,3,3-trifluoropropene (HCFO-1233xf), having low ozone depletion potential, is being considered as a replacement for chlorofluorocarbons such as dichlorodifluoromethane in refrigeration systems and trichlorofluoromethane as a blowing agent. The production of HFC's, i.e. compounds containing only carbon, hydrogen and fluorine has been the subject of interest to provide environmentally desirable products for use as solvents, blowing agents, refrigerants, cleaning agents, aerosol propellants, heat transfer media, dielectrics, fire extinguishing compositions and power cycle working fluids. It is known in the art to produce fluorocarbons such as HFC's by reacting hydrogen fluoride with various hydrochlorocarbon compounds. Such HFC's are not only considered to be much more environmentally advantageous than hydrochlorofluorocarbons (HCFCs) or chlorofluorocarbons (CFCs) because they are not non-ozone depleting, but also they are non-flammable, and non-toxic as compared to the chlorine containing compounds.

HCFO-1233xf is an intermediate in the production of 2,3,3,3-tetrafluoropropene (HFO-1234yf) which is well known in the art as described in U.S. Applications 20070007488 and 20070197842. HFO-1234yf has been disclosed to be an effective refrigerant, heat transfer medium, propellant, foaming agent, blowing agent, gaseous dielectric, sterilant carrier, polymerization medium, particulate removal fluid, carrier fluid, buffing abrasive agent, displacement drying agent and power cycle working fluid.

It has now been found that an important intermediate in the production of substantially pure HFO-1234yf, is an azeotropic or azeotrope-like composition of 2-chloro-3,3,3-trifluoropropene (HCFO-1233xf) and hydrogen fluoride. This intermediate, once formed, may thereafter be separated into its component parts by known extraction techniques. The azeotropic and azeotrope-like compositions find use not only as intermediates in the production of HFO-1234yf, but they are additionally useful as nonaqueous etchant mixtures for etching semiconductors in the electronics industry, as well as compositions for removing surface oxidation from metals. In addition, the formation of an azeotropic or azeotrope-like composition of HCFO-1233xf and hydrogen fluoride is useful in separating a mixture of HCFO-1233xf and an impurity such as a halocarbon, for example, 1,1,1,2,3-pentachloropropane; 1,1,2,3-tetrachloropropene; 2,3,3,3-tetrafluoropropene; 2,3-dichloro-3,3-difluoropropene; 1,1,1,2,2-pentafluoropropane; or 1,2-dichloro-3,3,3-trifluoropropene. When it is desired to separate a mixture of HCFO-1233xf and an impurity, HF is added to form an azeotropic mixture of HCFO-1233xf and hydrogen fluoride, and then the impurity is removed from the azeotropic mixture, such as by distillation or other known means. This binary azeotrope or azeotrope-like composition is then available for separation into its component parts.

EP 0 940 382 A1 discloses an azeotrope of HF and 1,1,1-trifluoro-3-chloro-2-propene.

WO 2008/054 781 A1 discloses azeotropic compositions of 2-chloro-3,3,3-trifluoro-1-propene with HF.

### SUMMARY OF THE INVENTION

The invention provides an azeotropic or azeotrope-like composition according to claim 1.

The invention also provides a method for removing 2-chloro-3,3,3-trifluoropropene from a mixture containing 2-chloro-3,3,3-trifluoropropene and at least one impurity according to claim 2.

### BRIEF DESCRIPTION OF THE DRAWING

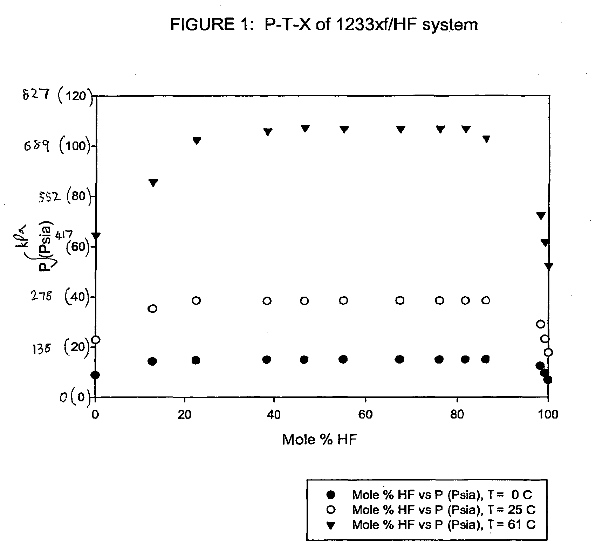

Figure 1 shows a plot of the vapor pressures of the mixtures formed in Example 2 as measured at 0 °C, 25 °C and 61 °C.

### DETAILED DESCRIPTION OF THE INVENTION

In a method of preparing an HCFO-1233xf precursor, reagents are fluorinated with hydrogen fluoride. This may be done, for example, by the gas phase catalytic fluorination of CCl₂=CClCH₂Cl with HF to yield HCFO-1233xf. Such methods are disclosed in U.S. Application 20070197842. The reaction products of such precursors include HCFO-1233xf, unreacted HF and other by-products. Upon removal of the by-products, a binary azeotrope or azeotrope-like composition of HCFO-1233xf and HF is formed. This binary azeotrope or azeotrope-like composition is then available for separation into its component parts. The azeotropic or azeotrope-like compositions of the HCFO-1233xf and HF are also useful as recycle to the fluorination reactor. Thus, for example, in a process for producing HCFO-1233xf, one can recover a portion of the HCFO-1233xf as an azeotropic or azeotrope-like composition of HCFO-1233xf and HF and then recycle the composition to the reactor.

HCFO-1233xf forms azeotropic and azeotrope-like mixtures with HF. The thermodynamic state of a fluid is defined by its pressure, temperature, liquid composition and vapor composition. For a true azeotropic composition, the liquid composition and vapor phase are essentially equal at a given temperature and pressure range. In practical terms this means that the components cannot be separated during a phase change. For the purpose of this invention, an azeotrope is a liquid mixture that exhibits a maximum or minimum boiling point relative to the boiling points of surrounding mixture compositions. An azeotrope or an azeotrope-like composition is an admixture of two or more different components which, when in liquid form under given pressure, will boil at a substantially constant temperature, which temperature may be higher or lower than the boiling temperatures of the components and which will provide a vapor composition essentially identical to the liquid composition undergoing boiling. For the purpose of this invention, azeotropic compositions are defined to include azeotrope-like compositions which means a composition that behaves like an azeotrope, i.e., has constant-boiling characteristics or a tendency not to fractionate upon boiling or evaporation. Thus, the composition of the vapor formed during boiling or evaporation is the same as or substantially the same as the original liquid composition. Hence, during boiling or evaporation, the liquid composition, if it changes at all, changes only to a minimal or negligible extent. This is in contrast with non-azeotrope-like compositions in which during boiling or evaporation, the liquid composition changes to a substantial degree. Accordingly, the essential features of an azeotrope or an azeotrope-like composition are that at a given pressure, the boiling point of the liquid composition is fixed and that the composition of the vapor above the boiling composition is essentially that of the boiling liquid composition, i.e., essentially no fractionation of the components of the liquid composition takes place. Both the boiling point and the weight percentages of each component of the azeotropic composition may change when the azeotrope or azeotrope-like liquid composition is subjected to boiling at different pressures. Thus, an azeotrope or an azeotrope-like composition may be defined in terms of the relationship that exists between its components or in terms of the compositional ranges of the components or in terms of exact weight percentages of each component of the composition characterized by a fixed boiling point at a specified pressure.

The present invention provides a composition which consists of from 79.3 to 85.3 mole percent of hydrogen fluoride and from 20.7 to 14.7 mole percent of HCFO-1233xf to form an azeotropic or azeotrope-like composition.

The composition of the present invention preferably has a boiling point of from 0 °C to 61 °C at a pressure of 103 kPa (15 psia) to 738 kPa (107 psia). In one embodiment it has a boiling point of 0 °C at a pressure of 103 kPa (15 psia). In another embodiment it has a boiling point of 25 °C at a pressure of 262 kPa (38 psia). In another embodiment it has a boiling point of 61 °C at a pressure of 738 kPa (107 psia). An azeotropic or azeotrope-like composition having 82.5 ± 1,2 mole percent HF and 17.5 ± 1.2 mole percent HCFO-1233xf was found at 25 °C.

In another embodiment of the invention, 2-chloro-3,3,3-trifluoropropene (HCFO-1233xf) may be removed from a mixture containing 2-chloro-3,3,3-trifluoropropene (HCFO-1233xf) and an impurity which may, for example, result from manufacturing steps in the preparation of 2-chloro-3,3,3-trifluoropropene (HCFO-1233xf). This is done by adding hydrogen fluoride to the mixture of the 2-chloro-3,3,3-trifluoropropene (HCFO-1233xf) and impurity. Hydrogen fluoride is added to the mixture in an amount sufficient to form an azeotropic composition of the 2-chloro-3,3,3-trifluoropropene (HCFO-1233xf) and the hydrogen fluoride, and thereafter the azeotropic composition is separated from the impurity, for example by distillation or other art recognized separating means. In one embodiment, the impurity itself does not form an azeotropic mixture with 2-chloro-3,3,3-trifluoropropene (HCFO-1233xf), hydrogen fluoride or a mixture of 2-chloro-3,3,3-trifluoropropene (HCFO-1233xf) and hydrogen fluoride. In another embodiment, the impurity does form an azeotropic mixture with 2-chloro-3,3,3-trifluoropropene (HCFO-1233xf), hydrogen fluoride or a mixture of 2-chloro-3,3,3-trifluoropropene (HCFO-1233xf) and hydrogen fluoride. Typical impurities of 2-chloro-3,3,3-trifluoropropene (HCFO-1233xf) include other halocarbons which may be miscible with 2-chloro-3,3,3-trifluoropropene (HCFO-1233xf) such as 1,1,1,2,3-pentachloropropane; 1,1,2,3-tetrachloropropene; 2,3,3,3-tetrafluoropropene; 2,3-dichloro-3,3-difluoropropene(HCFO-1232xf); 1,1,1,2,2-pentafluoropropane; or 1,2-dichloro-3,3,3-trifluoropropene.

### EXAMPLE 1

60 g of 2-chloro-3,3,3-trifluoropropene (HCFO-1233xf) were mixed with 40 g of HF to form a heterogeneous azeotrope mixture. The vapor pressure of the mixture at 25 °C was 262 kPa (38 psia).

### EXAMPLE 2

Binary compositions containing solely 2-chloro-3,3,3-trifluoropropene (HCFO-1233xf) and HF were blended to form a heterogeneous azeotrope mixtures at different compositions. The vapor pressures of the mixtures were measured at 0, 25 and 61 °C and the following results were noticed. Table 1 shows the vapor pressure measurements of HCFO-1233xf and HF as a function of composition with varying weight percent HF at constant temperatures of 0, 25, and 61°C. The data also showed that HCFO-1233xf/HF is a heterogeneous mixture.

**TABLE 1: P-T-X of HCFO-1233xf/HF**

| | Pressure kPa (Psia) | | |
|---|---|---|---|
| Mole% HF | T = 0 °C | T = 25 °C | T = 61°C |
| 0.00 | 61 (8.87) | 158 (22.88) | 445 (64.58) |
| Mole% HF | T = 0 °C | T = 25 °C | T = 61°C |
| 12.70 | 98 (14.21) | 243 (35.2) | 590 (85.62) |
| 22.50 | 101 (14.69) | 265 (38.48) | 706 (102.4) |
| 38.34 | 103 (15.03) | 265 (38.4) | 731 (106.08) |
| 46.46 | 103 (15.03) | 264 (38.35) | 740 (107.34) |
| 55.06 | 103 (15.03) | 265 (38.45) | 733 (106.95) |
| 67.39 | 103 (15.03) | 265 (38.45) | 733 (106.95) |
| 76.06 | 103 (15.03) | 265 (38.45) | 738 (107) |
| 81.72 | 103 (15.03) | 265 (38.45) | 735 (107.05) |
| 86.27 | 103 (15.08) | 265 (38.4) | 709 (102.88) |
| 98.36 | 86 (12.51) | 200 (29.04) | 500 (72.53) |
| 99.30 | 67 (9.7) | 160 (23.17) | 426 (61.72) |
| 100.00 | 47 (6.87) | 123 (17.82) | 361 (52.43) |

The data also shows that the mixture is azeotropic or azeotrope-like since the vapor pressure of the mixtures of HCFO-1233xf and HF is higher, at all indicated blend proportions, than vapor pressures of HCFO-1233xf and HF alone, i.e. as indicated in the first and last rows of Table 1 when HF is 0.0 mole % and HCFO-1233xf is at 100.0 mole % as well as when HCFO-1233xf is at 0.0 mole % and HF is at 100.0 mole %. The data from Table 1 is shown in graphic form in Figure 1.

### EXAMPLE 3

The azeotropic or azeotrope-like composition of the HCFO-1233xf /HF mixture was also verified by Vapor-Liquid-Liquid equilibrium (VLLE) experiment. 63.5 g of 2-chloro-3,3,3-trifluoropropene (HCFO-1233xf) were mixed with 36.5 g of HF to form a heterogeneous mixture (visual observation) at 24 °C. The vapor composition, upper liquid (HF rich), and bottom liquid (organic) were sampled. The result shows that the azeotropic composition is about 82.5 ± 1.2 mole percent HF at 24 °C.

## Claims

1. An azeotropic or azeotrope-like composition which consists of from 79.3 to 85.3 mole per cent of hydrogen fluoride and from 20.7 to 14.7 mole per cent of 2-chloro-3,3,3-trifluoropropene (HCFO-1233xf).

2. A method for removing HCFO-1233xf from a mixture containing HCFO-1233xf and at least one impurity, which comprises adding hydrogen fluoride to the mixture in an amount sufficient to form an azeotropic or azeotrope-like composition of HCFO-1233xf and hydrogen fluoride, and thereafter separating the azeotropic composition from the impurities, wherein the azeotropic composition consists of from 79.3 to 85.3 mole per cent hydrogen fluoride and 20.7 to 14.7 mole per cent HCFO-1233xf.

## Patentansprüche

1. Azeotrope oder azeotropartige Zusammensetzung, die aus 79,3 bis 85,3 Molprozent Fluorwasserstoff und 20,7 bis 14,7 Molprozent 2-Chlor-3,3,3-trifluorpropen (HCFO-1233xf) besteht.

2. Verfahren zur Entfernung von HCFO-1233xf aus einer Mischung, die HCFO-1233xf und mindestens eine Verunreinigung enthält, das die Zugabe von Fluorwasserstoff zu der Mischung in einer ausreichenden Menge, um eine azeotrope oder azeotropartige Zusammensetzung von HCFO-1233xf und Fluorwasserstoff zu bilden, und danach die Trennung der azeotropen Zusammensetzung von den Verunreinigungen umfasst, wobei die azeotrope Zusammensetzung aus 79,3 bis 85,3 Molprozent Fluorwasserstoff und 20,7 bis 14,7 Molprozent HCFO-1233xf besteht.

## Revendications

1. Composition azéotropique ou de type azéotropique qui consiste en de 79,3 à 85,3 pour cent en moles de fluorure d'hydrogène et de 20,7 à 14,7 pour cent en moles de 2-chloro-3,3,3-trifluoropropène (HCFO-1233xf).

2. Procédé permettant de récupérer le HCFO-1233xf à partir d'un mélange contenant le HCFO-1233xf et au moins une impureté, qui comprend l'addition de fluorure d'hydrogène au mélange en une quantité suffisante pour former une composition azéotropique ou de type azéotropique de HCFO-1233xf et de fluorure d'hydrogène puis la séparation de la composition azéotropique des impuretés, la composition azéotropique consistant en de 79,3 à 85,3 pour cent en moles de fluorure d'hydrogène et de 20,7 à 14,7 pour cent en moles de HCFO-1233xf.
